(19) Europäisches Patentamt
European Patent Office
Office européen des brevets

(11) **EP 4 549 934 A1**

(12) **EUROPEAN PATENT APPLICATION**

(43) Date of publication:
**07.05.2025 Bulletin 2025/19**

(21) Application number: **23206767.8**

(22) Date of filing: **30.10.2023**

(51) International Patent Classification (IPC):
**G01N 33/487** $^{(2006.01)}$      **C12Q 1/6869** $^{(2018.01)}$
**C12Q 1/6874** $^{(2018.01)}$

(52) Cooperative Patent Classification (CPC):
**G01N 33/48721**

---

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Adolphe Merkle Institute, University of Fribourg**
**1700 Fribourg (CH)**

(72) Inventors:
• **Mayer, Michael**
  **3186 Duedingen (CH)**

• **Ianiro, Alessandro**
  **3020 Herent (BE)**
• **Chanakul, Wachara**
  **1700 Fribourg (CH)**
• **Mukhopadhyay, Anasua**
  **1700 Fribourg (CH)**

(74) Representative: **Stolmár & Partner Intellectual Property GmbH**
**Bahnhofstrasse 106**
**8001 Zürich (CH)**

Remarks:
The complete document including Reference Table(s) and the Sequence Listing(s) can be downloaded from the EPO website

---

(54) **C9 NANOPORES**

(57)     The invention relates to a nanopore-based sensing device comprising at least one membrane, the membrane comprising at least one nanopore comprising complement component 9 (C9) monomers.

**EP 4 549 934 A1**

## Description

### Field of the invention

The invention relates to the field of nanopore-based sensing and sensing devices based on biological nanopores. These sensors can be used for analyzing biopolymers and nanoparticles.

### Background

Sensing technology based on biological nanopores has become an increasingly important analytical tool as it allows the characterization of unlabeled synthetic and biological molecules such as polymers, DNA, proteins, peptides and small molecular analytes. Biological nanopore sensors typically consist of two electrolyte-filled compartments (called cis- and trans-compartments, respectively) separated by a phospholipid membrane bearing a single nanometric pore. When a potential difference is applied across the membrane, the electrolyte ions flow through the nanopore, resulting in a net ionic current. The translocation or entry of a (macro)molecule through the pore hinders the passage of ions, resulting in transient decrease of the ionic current. This signal, called resistive pulse, can be analyzed in real-time or offline to determine structural properties of the analyte.

If the nanopore diameter is larger than the largest dimension of the translocating molecule, resisting pulse sensing provides information on the volume and shape of the analyte. To reliably determine volume and shape, a protein has to sample different orientations while translocating through the nanopore. Biological nanopores employed in resistive pulse sensing are typically too small for volume or shape determination. The largest biological nanopore to date is a two-component pleurotolysin (PlyAB) toxin, which can form cylindrical pores with an internal diameter of 5.5 nm and length of 10 nm. Such a pore enables the detection of small folded proteins up to approximately 64.5 kDa, while translocation of larger proteins such as human albumin (HSA) and transferrin (HTr) necessitates specific engineering of the lumen of the PlyAB pore. Solid-state nanopores can be used as an alternative to biological nanopores for determining volume and shape of folded proteins within a size range of approximately 50 to 970 kDa. Nevertheless, solid-state nanopores have several drawbacks. First, proteins tend to absorb onto the inner surface of the nanopore. Second, achieving uniformity in the manufacturing of solid-state nanopores is crucial for reliable detection yet is still challenging. Third, compared to biological nanopores, modifying the internal pore surface to optimize detection is not straightforward: the surface charge, which governs the nanofluidic properties of the pore, cannot be modified with atomic precision, and binding elements cannot be introduced with controlled stoichiometry. Fourth, solid-state nanopores often grow in diameter due to slow etching of the material that bears the pore. This etching changes the shape of the pore as well as its size, thereby introducing uncertainty in the structural characterization of translocating macromolecules.

Because of the limitations of solid-state nanopores, the development of large biological nanopores enabling volume and shape determination of a wide range of proteins is crucial.

### Objective technical problem to be solved

There is therefore a need in the art for large-diameter biological nanopores that can be used to determine volume and shape of proteins and other nanoparticles.

### Summary of the invention

### Brief description of the figures

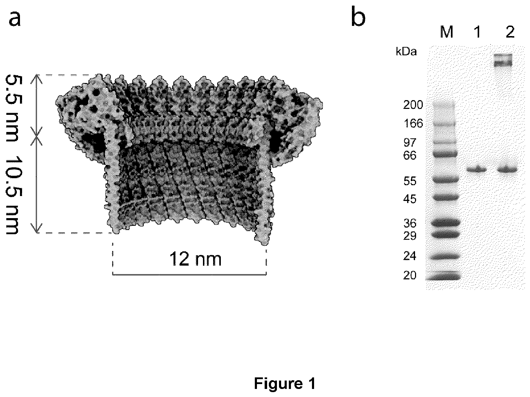

Figure 1 shows the Cross-section of a polyC9 assembly and its characterization through SDS-PAGE gel electrophoresis. a) Cross-sectional view of the structure of polyC9 determined by cryogenic electron microscopy (PDB ID: 5fmw). b, Sodium dodecyl sulfate - polyacrylamide gel electrophoresis (gel strength: 4 - 15%) profile of C9 prior (lane 1) and after (lane 2) overnight incubation at 37 °C in 10 mM HEPES, 50 mM NaCl (pH = 7.5) buffer containing 0.02 mg·ml$^{-1}$ amphipol. Lane M contains the standard markers.

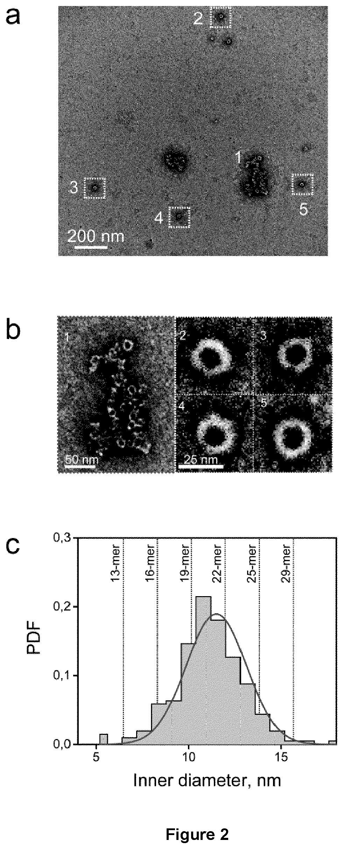

Figure 2 shows the characterization of the polyC9 pore assembly through transmission electron microscopy (TEM). a, b) TEM micrographs of polyC9 assemblies stained with uranyl acetate. c) Histogram representing the inner diameters of polyC9 pore calculated from the analysis of TEM images using imaged (N = 205). The vertical dotted lines represent theoretical predictions of the pore diameter for various polyC9 assemblies.

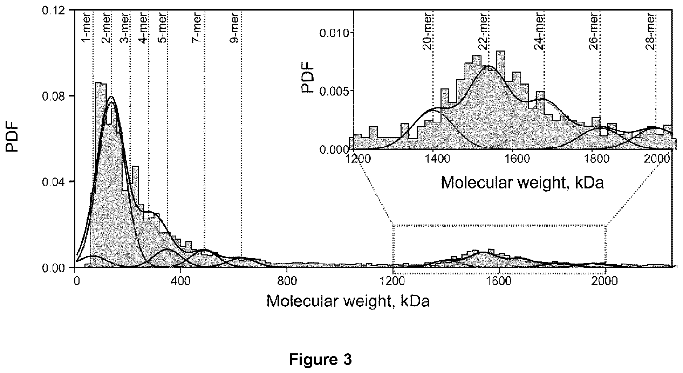

**Figure 3** shows the characterization of the polyC9 pore assembly through single particle mass photometry analysis. Mass photometry distribution of C9 incubated overnight at 37 °C in 10 mM HEPES, 50 mM NaCl (pH = 7.5) buffer containing 0.02 mg·ml⁻¹ amphipol. The inset shows a zoomed-in region corresponding to the expected mass range of polyC9. The vertical dotted lines represent the expected mass of various polyC9 assemblies.

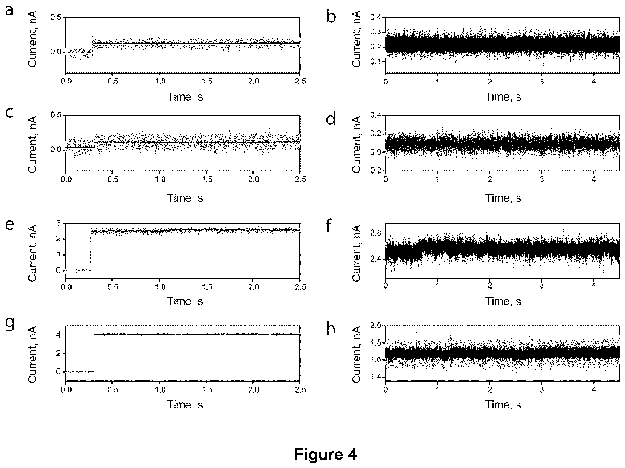

**Figure 4** shows insertions of native and cross-linked polyC9 nanopores into planar lipid bilayers at various ionic strengths. Panels a, c, e, g show polyC9 single-pore insertions into lipid bilayer membranes, while panels b, d, f, h displays the corresponding open-pore currents. (a, b) polyC9 insertion at 50 mM NaCl; (c, d) crosslinked polyC9 insertion at 50 mM NaCl; (e, f) polyC9 insertion at 1 M NaCl; (g, h) insertion at 1 M NaCl of polyC9 in a recording buffer containing 0.02 mg·ml⁻¹ amphipol. In all panels, the applied potential was +100 mV, and the recording buffer consisted of 10 mM HEPES, pH 7.5 plus the indicated salt concentration. The current recordings were collected with a 200 kHz sampling rate (gray traces). The recordings in panels a, c, e and g were filtered with a 1 kHz low-pass Gaussian filter (black traces). The recordings in panels b, d, f and h were filtered with a 20 kHz low-pass Gaussian filter (black traces).

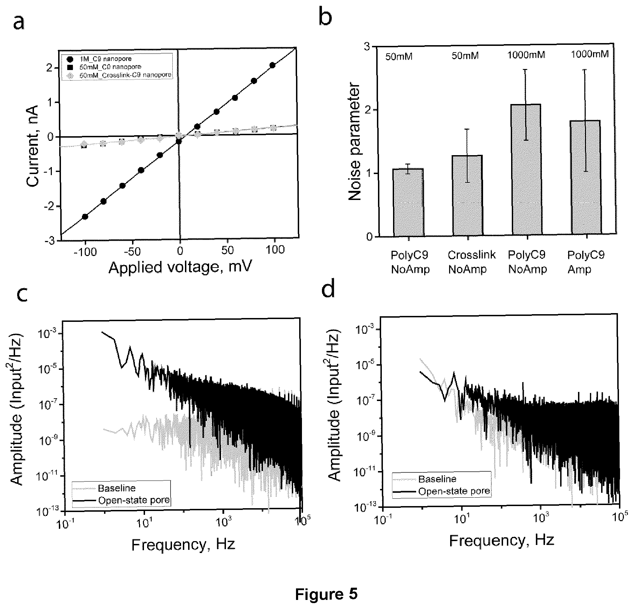

**Figure 5** shows the electrophysiological characterization of native and cross-linked polyC9 pores in various recording buffers. a) Comparison of the current-voltage (I-V) curves of C9 nanopore at 50 mM NaCl (squares), chemically crosslinked C9 nanopore at 50 mM NaCl (diamonds) and 1 M NaCl (circles). b) Noise parameter calculated as the standard deviation of the open pore current divided by the standard deviation of the baseline current for C9 nanopores under various conditions. The error bars represent the standard deviation of the mean. c, d) Power spectral densities (PSD) of the open-pore currents of C9 nanopores at 1 M NaCl (c) in the absence and (d) in the presence of Amphipol. In all panels the applied potential was +100 mV and the recording buffer consisted of 10 mM HEPES at pH 7.5 plus the indicated salt concentration.

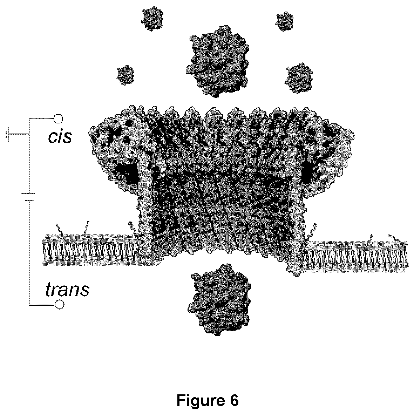

**Figure 6** shows the schematic of a recording setup for single molecule analysis with C9 nanopore embedded in a planar lipid bilayer.

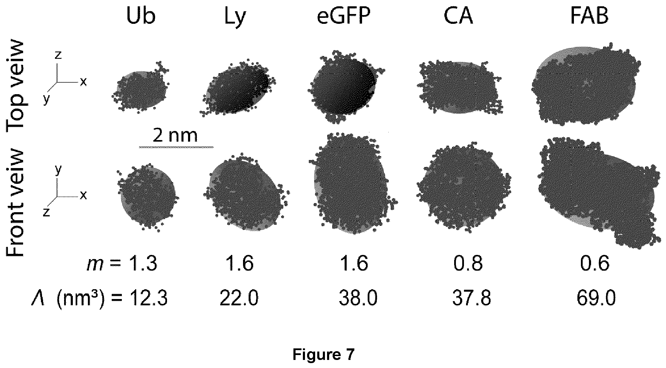

**Figure 7** shows the shape approximation determined by modelling the crystal structure of five proteins from the Protein Data Bank (Ub, 1ubq; Ly, 1dpx; eGFP, 2y0g; CA, 2cab; FAB, 7fab, dots) with ellipsoids of rotation having the same volume of the proteins (grey spheroids).

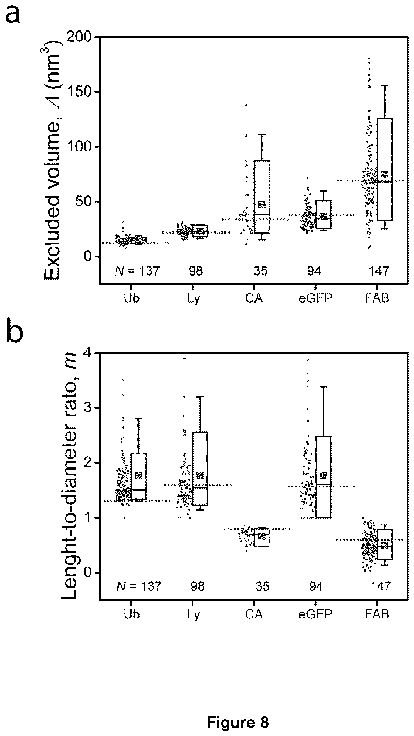

**Figure 8** shows the estimation of protein volume and length-to-diameter ratio of analytes translocating through the C9 nanopores. a) Distribution of excluded volumes ($\Lambda$) and b) length-to-diameter ratios ($m$) determined from individual resistive pulses from the translocation of five different proteins. For each sample in both panels, the filled squares represent the mean, the continuous horizontal line represent the median, the box range represents the 25th to 75th percentile and the whisker range represents the 10th to 90th percentile. Horizontal dotted lines are the theoretical reference values.

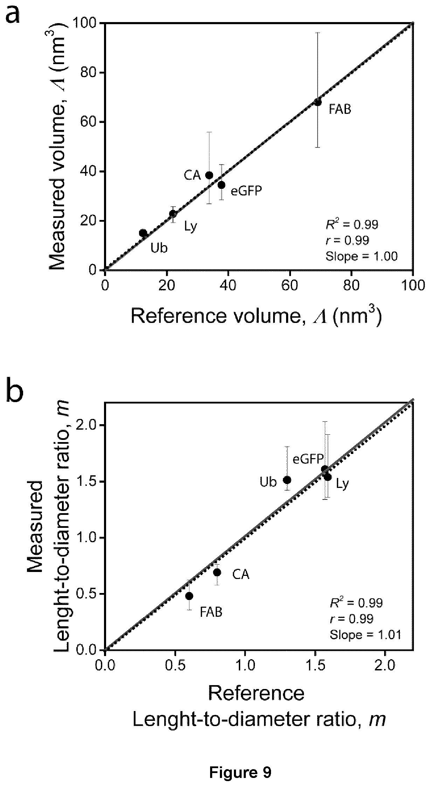

**Figure 9** shows the comparison of the estimated volumes and length-to-diameter ratios of five proteins with the expected theoretical values. Median values of (a) the excluded volume ($\overline{\Lambda}$), and (b) the length-to-diameter ratio ($\overline{m}$) determined from single event analyses of five proteins plotted against the expected theoretical values. Error bars show first and third quartiles. The black dotted lines represent the ideal 1:1 agreement (slope = 1), and the solid lines are the linear regressions performed imposing a zero intercept.

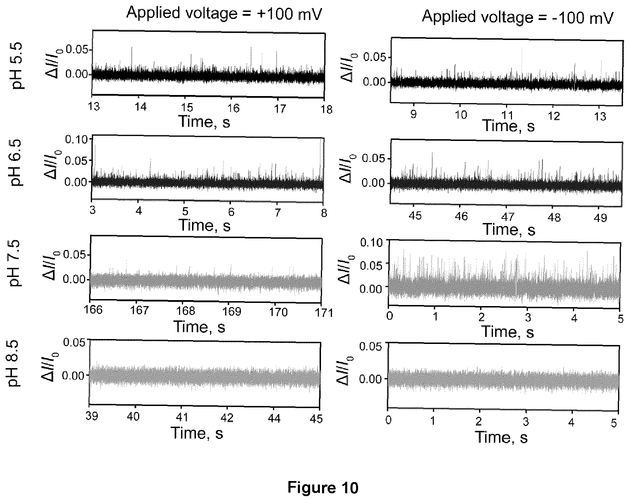

**Figure 10** shows the translocation of ubiquitin through the C9 nanopore at various pH. The normalized current traces on the left are recorded with an applied potential difference of +100 mV. The normalized current traces on the right are recorded with an applied potential different of -100 mV. Upward spikes correspond to the translocation of a single protein. A Python script was used to perform baseline correction and normalize the current recordings.

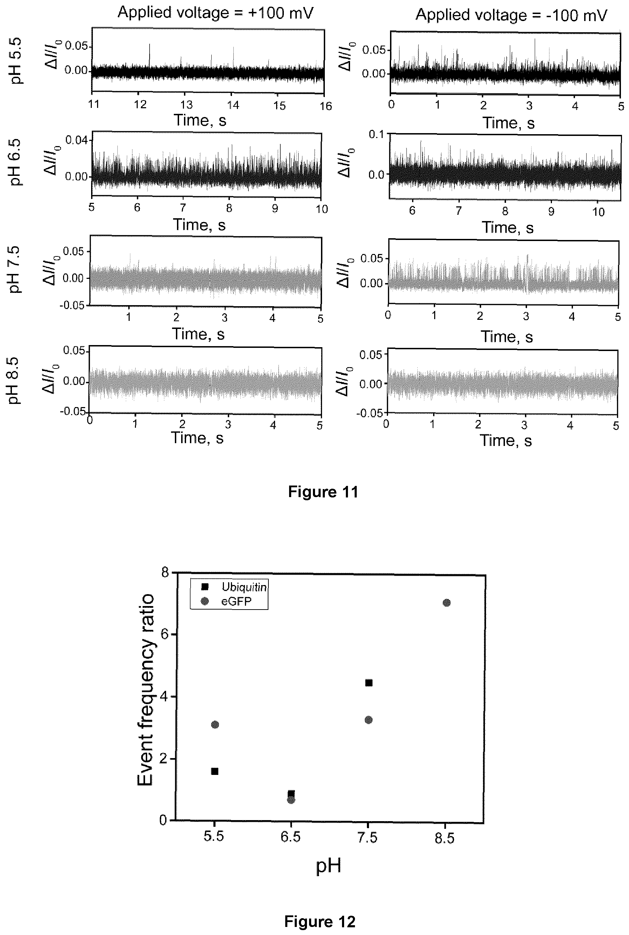

**Figure 11** shows the translocation of eGFP through the C9 nanopore at various pH. The normalized current traces on the left are recorded with an applied potential difference of +100 mV. The normalized current traces on the right are recorded with an applied potential different of -100 mV. Upward spikes correspond to the translocation of a single protein. A Python script was used to perform baseline correction and normalize the current recordings.

**Figure 12** shows the translocation event frequency ratios of ubiquitin and eGFP through the C9 nanopore at various pH. The event frequency ratio was calculated by dividing the event frequency at -100 mV applied voltage by the event frequency at +100 mV applied voltage.

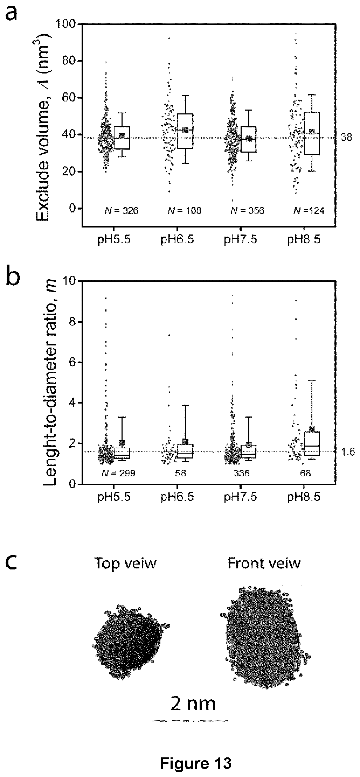

**Figure 13** shows an individual event analysis of eGFP translocated through C9 nanopores at various pH. a) Excluded

volume, $\Lambda$, and b) length-to-diameter ratio m of eGFP determined from individual translocation events at various pH. For each sample in a) and b), the filled squares represent the mean, the continuous horizontal line represent the median, the box range represents the 25th to 75th percentile and the whisker range represents the 10th to 90th percentile. The expected theoretical values, calculated from the crystal structures of the proteins (PDB: 2Y0G), are indicated by horizontal dotted lines. c) Comparison between the approximate shape and volume of eGFP determined by analyzing individual resistive pulses (gray spheroids) and crystal structure of the protein (dots).

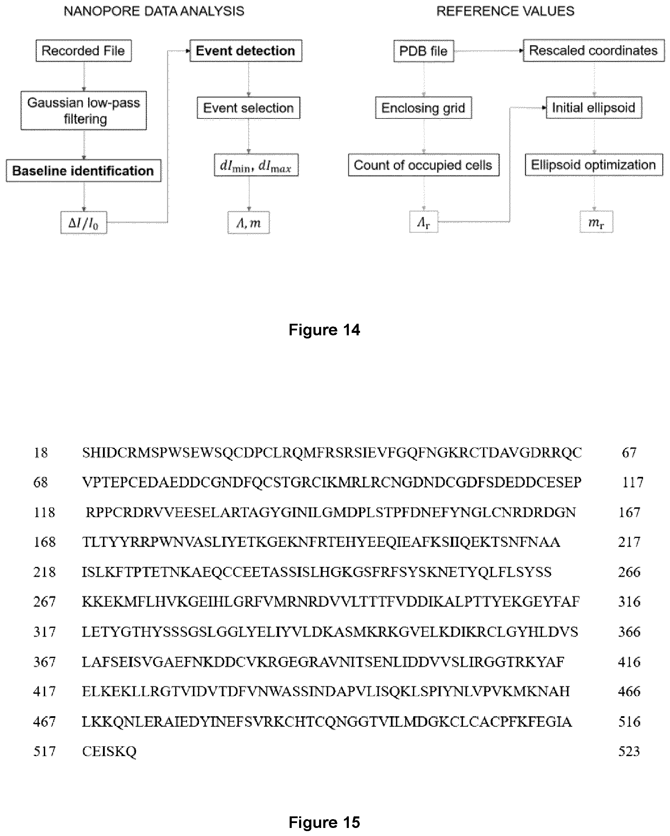

**Figure 14** shows the schematic representation of the procedure employed for analyzing the nanopore data and calculating the reference volume and shape from the atomic coordinates of the target analyte.

**Figure 15** shows the amino acid sequence of C9 monomer *(human,* Chain A; PDB: 5fmw). Amino acids inside the pore lumen (Y183 to H281 and S330 to I408), as well as amino acids located at the region on top of the pore (C80 to T134 and S148 to I182), or at the side region of top (S18 to T70 and S485 to Q523).

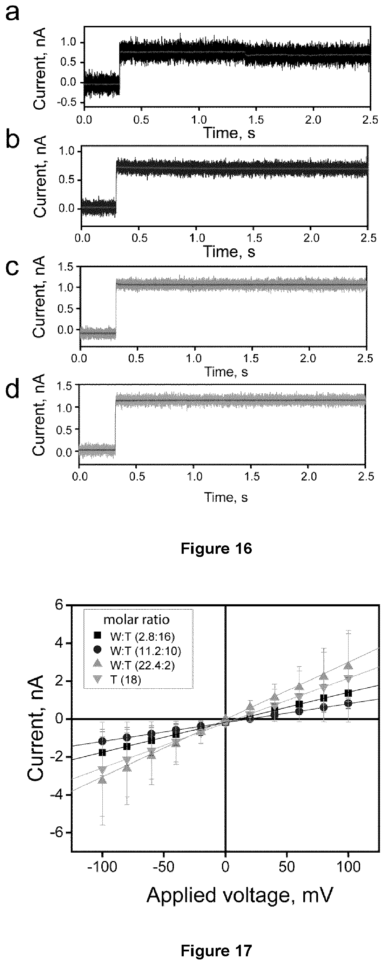

**Figure 16** shows the electrophysiological characterization of pores formed by mixing in wild-type C9 with a truncated version of C9 that is missing the last 20 amino acids at N-terminus. The samples were prepared by mixing three stock solutions in different proportions. The stock solution of wild-type C9 monomer (W) had a concentration of 2 mg/ml in 50 mM M NaCl, 10 mM HEPES pH 7.5. The stock of truncated C9 (T) had a concentration 1.34 mg/ml in 150 mM M NaCl, 10 mM PBS pH 7.2. The third stock consisted of a 0.02 mg/ml Amphipol (A) solution in 50 mM M NaCl, 10 mM HEPES pH 7.5. The three stocks were mixed to obtain a final volume of 10 $\mu$L and incubated overnight. In all the final solutions, Amphipol concentration were maintained to 0.002 mg/ml. The incubated solutions were added to a recording buffer containing of 1 M NaCl, 10 mM HEPES pH 7.5, 0.002 mg/ml Amphipol to study polyC9 insertion into lipid bilayers. (a) Current trace showing polyC9 insertion at a molar ratio of W : T of 2.8 : 16; (b) polyC9 insertion at the ratio of W : T of 11.2 : 10; (c) polyC9 insertion at the ratio of W : T of 22.4 : 2; (d) polyC9 insertion at 18 $\mu$M) of T. In all panels the applied potential was +100mV and the recording buffer. The current recordings were collected with an Orbit mini (Nanion) using a sampling frequency of 200 kHz (dark gray traces) and are also displayed after filtering with a 1 kHz low-pass Gaussian filter (light grey traces).

**Figure 17** shows the comparison of the current-voltage (I-V) curves of pores formed by mixing in different proportions wild-type C9 with a truncated version of C9 that is missing the last 20 amino acids at N-terminus. Error bars represent the standard deviations calculated from a minimum of three repeats.

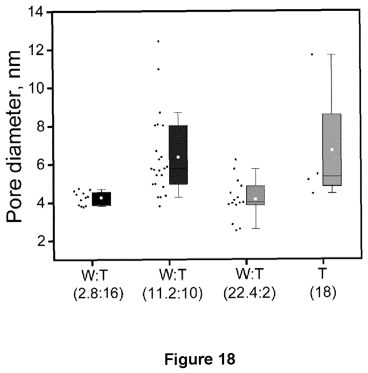

**Figure 18** shows the estimated inner diameters of pores formed by mixing in different proportions wild-type C9 with a truncated version of C9 that is missing the last 20 amino acids at N-terminus. The mean values are represented by white squares. The box range corresponds to the 25th to 75th percentile, whereas the whisker range corresponds to the 5th to 95th percentile.

## Detailed description

In one aspect, the invention relates to a nanopore-based sensing device comprising at least one membrane, the membrane comprising at least one nanopore comprising or consisting of complement component 9 (C9) monomers.

A nanopore-based sensing device or nanopore sensor is herein defined as a system, e.g., a flow cell, comprising at least one membrane, the membrane comprising at least one nanopore, that is capable of detecting the presence and/or determing the volume and shape of an analyte. The nanopore-based sensing device derives its sensing capability from the nanopore used therein, wherein the nanopore allows the translocation of the analyte.

The nanopore-based sensing device may comprise additional elements such as chambers, scaffolds, electrodes, circuits, sensor chips, liquid medium, stabilizers or buffers that are necessary to support and stabilize the membrane and/or the nanopore and to enable the generation and read-out of an electric signal.

The nanopore-based sensing device according to the invention is capable of sensing proteins, e.g., native or denatured proteins, (bio)macromolecules, e.g., DNA or RNA molecules including DNA origami, exosomes or liposomes, nanoparticles and assemblies of nanoparticles and colloids.

In a preferred embodiment, the sensing properties, e.g., translocation frequency, residence time in the pore, wall interactions, of the nanopore-based sensing device are adjustable via the applied electric field, properties of the ions in the electrolyte solution, concentration of ions in the electrolyte solution, and/or the pH of the electrolyte solution. The translocation of proteins through nanopores takes place under the influence of two main forces: electrophoresis and hydrodynamic drag by electroosmotic flow. These phenomena depend on the applied potential V, the charge of the proteins, the charge of the inner surface of the nanopore, and the concentration of ions. The charge state of a protein, as well as that of a surface, depends on the nature of the charged groups, the pH of the medium and the concentration of the ions. Without wanting to be bound by a mechanism, it is believed that for the C9 nanopores of the invention, selecting a specific pH value, specific ionic strength, as well as a specific applied potential make it possible to control the balance between electrophoresis and electroosmotic flow, thereby favoring or disfavoring the translocation of a certain analyte of interest versus other molecules or analytes in solution.

The membrane in the nanopore-based sensing device according to the invention may be any membrane used in the art for embedding a biological nanopore. In a preferred embodiment, the membrane is a planar lipid bilayer membrane. In another preferred embodiment, the membrane is a block-copolymer membrane.

According to the invention, the at least one membrane of the nanopore-based sensing device comprises at least one nanopore. In some embodiments, the membrane comprises exactly one nanopore or several nanopores. In some embodiments, the membrane divides a chamber into two compartments, with the nanopore being the only connection between the two compartments.

In a preferred embodiment, the nanopore-based sensing device according to the invention comprises a multitude of membranes each comprising exactly one nanopore.

The nanopores used in the nanopore-based sensing device according to the invention comprise complement component 9 (C9) monomers. C9 is a protein involved in the complement system and thus the innate immune system. C9 represents one of the main components of the complement membrane attack complex (MAC) and is capable of forming pores together with other complement proteins such as C6, C7, C8 and C5b.

Naturally occurring, wild-type human C9 monomer has the amino acid sequence depicted in SEQ ID NO:1. As used herein, the term C9 monomer comprises not only proteins or polypeptides having exactly the sequence of SEQ ID NO: 1, but also proteins or polypeptides having a sequence that is at least 80%, at least 85%, at least 90%, at least 95%, at least 97%, at least 98% or at least 99% identical to SEQ ID NO: 1, the identity being determined over the whole length of the sequence by any method known in the art. The C9 monomers may also be shortened or lengthened compared to the wild-type C9 monomer.

Preferably, the nanopores used in the nanopore-based sensing device according to the invention are made up of and consist entirely of C9 monomers. In some embodiments, the nanopore comprises between 10 and 35 C9 monomers. In preferred embodiment, the nanopore is an assembly of and thus comprises 22 C9 monomers.

In a preferred embodiment, the nanopores used in the context of the invention are cylindrically shaped.

In some embodiments, the nanopores have an inner diameter in the range of 5 to 15 mm. In a preferred embodiment, the nanopores have a diameter of about or exactly 10 nm.

The nanopore may diameter may be calculated by calculating the equivalent pore diameter of a cylindrical pore based on the conductance values of the difference between baseline current (V = +100 mV) and the current after single-pore insertion of polyC9 (V = +100 mV), taking into account the channel and access resistances.

*Determination of the inner diameter with the following equations:*

$$d_\mathrm{p} = \frac{\rho_\mathrm{el}\, G}{\pi} \times \left( \frac{\pi}{2} + \sqrt{\frac{\pi^2}{4} + \frac{4\pi l_\mathrm{p}}{\rho_\mathrm{el}\, G}} \right) \tag{1}$$

Where;

$d_\mathrm{P}$ (m) = Inner diameter of nanopore
$\rho_\mathrm{e1}$ (Ω.m) = Electrical resistivity of the electrolyte buffer
$G$ (Ω$^{-1}$) = Conductance difference between baseline and open-state pore current
$l_\mathrm{P}$ (m) = Effective pore length of 13 nm.

Then the number of C9 monomers within C9 nanopores using the geometric model is calculated.

*Determination of the inner diameter with the following equations:*

$$d_p = s \sqrt{\frac{1}{\pi} \left( \frac{n}{tan\left(\frac{\pi}{n}\right)} - \frac{(n-2)\pi}{2} \right)} \tag{2}$$

Where;

$d_\mathrm{P}$ (m) = Inner diameter of the nanopore, taking these values from equation (1)

η = Number of monomers in C9 nanopore

s = Diameter of a C9 monomer in C9 nanopore the diameter of the cylinder was is calculated based on equation (2) where the inner diameter of the nanopore is 12 nm and polyC9 pore consists of 22 C9 monomers. The diameter of a C9 monomer in a C9

nanopore is 1.93 nm.

In some embodiments, the nanopores have an effective length in the range of 3.5 to 20 nm. In a preferred embodiment, the nanopores have an effective length of about or exactly 13 nm.

The nanopores used in the context of the invention have a larger internal diameter than any other biological nanopore previously described. This makes them particularly useful for determining the volume and shape of large analytes such as proteins, macromolecules and nanoparticles since the larger the nanopore, the larger the analyte that can be analyzed with the help of said nanopore. For example, analytes with a size of up to 10 nm can be analyzed.

In addition, the nanopores used in the context of the invention are capable of self-incorporating into a membrane and form stable pores over a wide range of pH and ionic strengths that remain stable over a long period of time. Their easy assembly make s the nanopores of the invention ideal for the use in nanopore-based sensing devices.

Furthermore, the nanopores do not show gating, i.e., no spontaneous opening and closing of the pore, or clogging, i.e., no permanent current reduction due to proteins sticking to the lumen wall.

The nanopores of the invention have a cylindrical pore shape, which is favorable as the electric field inside the pore is constant along its length making it possible to use modulations in current due to rotations of an analyte to determine its shape. Additionally, their effective length is also comparably long, making it suitable for the determination of details of shape, volume, dipole moment, etc. For the discussion of mutations in C9 subunits for use in the invention herein below, the numbering of the sequence as shown in Fig. 9 is adhered to. This numbering corresponds to the residue numbering in naturally occurring human C9 of SEQ ID NO: 1.

In some embodiments, at least one C9 monomer used in the nanopores of the Nanopore-based sensing device according to the invention is modified compared to a naturally occurring monomer, the modification being an amino acid exchange, deletion and/or insertion and/or a chemical derivatization of an amino acid.

The term "chemical derivatization of an amino acid" is herein defined as any chemical modification that maintains the basic chemical structure and properties of the amino acid. Examples thereof include addition of functional groups such as maleimides or hydroxysuccinimide (NHS) to amine or thiol groups or modification of carboxylic groups, e.g., by N-ethyl-3-N', N'-dimethyl aminopropyl carbodiimide (EDC) coupling.

In some embodiments, at least one C9 monomer is modified by replacing at least one cysteine residue with another amino acid and/or by adding a functional group to at least one cysteine residue. In preferred embodiments, cysteines between Y183 to H281, S330 to I408, C80 to T134 and/or S148 to 1182 are modified.

In a preferred embodiment, all cysteine residues are replaced with other amino acids, preferably an amino acid that does not affect the pore-forming function of the protein, e.g., alanine. In other embodiments, at least one cysteine residue is modified by adding a functional group such as an azide group or a maleiimde. Addition of functional groups allows for the attachment of further functional groups, e.g., nanobodies capable of recognizing particular analytes.

Cysteine residues generally show a low abundance and have the highest nucleophilicty of all naturally occurring amino acides. By replacing and/or modifying individual or all cysteine residues, a high selectivity of functionalization is therefore achieved.

In some embodiments, at least one C9 monomer is modified by replacing at least one amino acid carrying a charge with an amino acid carrying the opposite or no charge. Amino acids carrying a negative charge at pH 7 are aspartic acid and glutamic acid.

Amino acids carrying a positive charge at pH 7 are lysine, arginine and histidine. At pH 7.5, the lumen of the C9 nanopores used in the invention is negatively charged. This allows negatively charged proteins to translocate from the cis to the trans compartment under negative applied potential via the electroosmotic flow correction (EOF) as the dominating force. Changing the charge of the amino acids within the pore lumen changes the EOF and the electrophoretic force (EPF) and thus allows the translocation of highly negatively charged analytes or capture of large protein analytes (with a size of > 10 nm) through the nanopore.

In preferred embodiments, at least one amino acid selected from the group comprising residues 183 to 281 and 330 to 408 of SEQ ID NO: 1 is modified. These amino acids are inside the pore lumen of the C9 nanopores described herein. Modifying these amino acids allows to change and adjust the EOF as well as the electrostatic properties of the nanopore. This may be used to either attract certain analytes or to prevent them from entering the pore.

In another preferred embodiments, at least one amino acid selected from the group comprising residues 18 to 70, 80 to 134, 148 to 182, and 485 to 523 of SEQ ID NO: 1 is modified. These amino acids are outside of the membrane bound regions of the C9 nanopores described herein. Modifying these amino acids therefore allows to attach a functional group before the lumen of the nanopore, regions, e. g., an antibody for selective detection of its antigen.

In one embodiment, a C9 monomer to be used in the nanopore of the nanopore-based sensing device comprises modified amino acids both inside the pore lumen and outside the membrane bound region.

In some embodiment, the C9 monomer is truncated from the N-terminus. This enhances pore formation. In a preferred embodiment, 16 or 20 amino acids from the N-terminus of the C9 monomer are deleted.

In another preferred embodiment, the C9 monomers are crosslinked with each other.

In addition to the mutations described above, the C9 monomers for use in a nanopore-based sensing device according to the invention may comprise one or more conservative mutations. Typically, conservative mutations wherein an amino acid is replaced by a residue with very similar properties are anticipated to have no or only a limited effect on nanopore function. Examples of conservative mutations include S to T, R to K, D to E, N to Q, A to V, I to L, F to Y and vice versa.

In some embodiments, only some C9 monomers of the nanopore are modified. By mixing wild-type C9 monomers and modified monomers with each other, it is possible to e.g., selectively introduce individual binding sites into the pore lumen.

In other embodiments, all C9 monomers of the nanopore are modified.

In some embodiments, the C9 monomers carry different mutations. In other embodiments, the C9 monomers carry the same mutation or mutations.

In some embodiments, the at least one nanopore of the nanopore-based sensing device is stabilized with a polymeric surfactant. In other embodiments, the at least one nanopore of the nanopore-based sensing device has been assembled in the presence of a polymeric surfactant. The polymeric surfactant is preferably an amphipol. Amphipols are a class of surfactants that substitute detergents and keep membrane proteins soluble in detergent-free aqueous solution by stabilizing them biochemically. Amphipols are commercially available, for example Amphipol A8-35 (Anatrace, OH).

In another aspect, the invention relates to a kit for assembling a nanopore-based sensing device, the kit comprising

- a device comprising at least one membrane,

- pre-conditioned C9 monomers and/or at least pre-assembled nanopore consisting of C9 monomers,

- means to facilitate assembly of the pre-conditioned C9 monomers into a nanopore and/or insertion of the assembled or pre-assembled nanopore into the membrane.

In one embodiment, the C9 monomers of the kit are pre-conditioned. Pre-conditioned as used herein means that the C9 monomers have been purified and/or solubilized with a polymeric surfactant. In another embodiment, the kit comprises a pre-assembled nanopore comprising or consisting of C9 monomers. The pre-assembled nanopore may be stabilized.

The means to facilitate assembly of the pre-conditioned C9 monomers into a nanopore and/or insertion of the assembled or pre-assembled nanopore into the membrane may be a buffer comprising further stabilizers. In one embodiment, the means are a buffer comprising a polymeric surfactant, preferably amphipol.

The kit of the invention allows a user to assemble a C9 nanopore-based sensing device ad hoc. This circumvents the problem of having to store the nanopore-based sensing device for a prolonged time which requires stabilizing the nanopore and the membrane.

In another aspect, the invention relates to the use of a nanopore comprising or consisting of C9 monomers for sensing proteins, macromolecules, nanoparticles and/or assemblies of nanoparticles. In a preferred embodiment, the nanopore is used for determining the volume and/or shape of a protein, macromolecule, nanoparticle and/or assembly of nanoparticles. For example, the nanopore can be used to detect particular antibodies, differentiate between single nanoparticles and nanopore assemblies or verify DNA origami structures.

In yet another aspect, the invention relates to the use of a nanopore comprising or consisting of C9 monomers for controlling the translocation probability of a molecule or particle of interest, i.e., favoring or disfavoring the translocation of a certain analyte of interest. Therefore, a nanopore comprising or consisting of C9 monomers may be used sorting and filtering analytes. The use may comprise tuning pH and/or applied potential or attaching particles to the nanopore.

In another aspect, the invention relates to a computer-implemented method of determining the volume and/or shape of an analyte, the method comprising steps a) to g).

In step a), a nanopore signal recording is recorded, wherein the signal is the current through the nanopore in the presence of the analyte, wherein the analyte is able to translocate through the nanopore and wherein a translocation event is the portion of the signal pertaining to the translocation of the analyte. The term "translocate" is herein defined as meaning that the analyte enters or passes through the nanopore.

In step b), a dynamic baseline trace is determined by processing the imported recording with an algorithm that discriminates between fluctuations of the signal caused by naturally occurring variations of the nanopore size and translocation events and using the dynamic baseline to normalize the imported signal.

In step c) a threshold-based search of the normalized signal is performed in order to identify translocation events.

In step d), the size of the nanopore at any time point in the recording from the dynamic baseline trace determined in step b) is calculated. This information enables a more precise determination of the volume of the analyte from the analysis of the translocation events when the size of the nanopore fluctuates over time.

In step e), each translocation event detected in step c) is analyzed with a percentile-based method that extracts the noise-corrected minimum and maximum amplitudes of the translocation event. This step results in the method avoiding fitting procedures, making it fast and suitable for inline analysis.

In step f), the volume of the analyte using the minimum and maximum amplitudes calculated in step e) and the actual nanopore size calculated in step d) is determined for each translocation event.

In step g), for each translocation event, the most probable shape of the analyte from the shape of the amplitude distribution of the translocation event and from the minimum and maximum amplitudes calculated in step e) is derived for each translocation event. The shape may be prolate or oblate.

The inventors have developed a data analysis method to obtain $\Lambda$ (excluded volume) and m (length-to-diameter ratio) from nanopore translocation event that accounts for fluctuations in the diameter of C9 nanopores during the recording, as well as a software to determine reference values for these two parameters form the atomic coordinates (.PDB file) of the target analyte. An embodiment of the method is schematically depicted in Fig. 8 and detailed below. This method is specifically tailored to distinguish between oblate and prolate particles. To determine the volume and shape, the shape of each protein is approximated as an ellipsoid of rotation with the three axes among which two are the same, i.e., ellipsoid axes: A, B, B. For the shape of the proteins, the m value is considered to define the shape of proteins (m<1 = oblate (lentil-shaped), m = sphere, m>1 = prolate (rugby-ball-shaped)). The shape of proteins m is approximated with the axis ratio of a spheroid: m = A/B.

*Detection and analysis of translocation events*

The procedure to detect and analyze translocation events from nanopore recordings consists of several sequential steps.

<u>Baseline</u> search. In the first step, the recording $x$, composed of $W$ samples, is imported and digitally filtered using a gaussian low-pass filter. The filtered data are subsequently processed with a custom-made baseline search algorithm that operates as follows:

1) the filtered recording $x^f$, with size $W$, is subdivided into $N + 1$ segments, $N$ containing $M$ samples each and one containing the remaining $W - NM$ samples. The value of $M$ is set by the user and should be always M >100 and at least 30 times longer than the longest translocation event.

2) An empty array $x^b$ with length $W$ is initialized, which will be filled with the estimated baseline values. For clarity, the $j^{th}$ element of an array x is indicated as $x[j]$,

3) The mean ($\overline{y}_i$) and the standard deviation ($\sigma_i$) of each segment $y_i$ is calculated.

4) The minimum standard deviation $\sigma_{min}$ is identified as the minimum value among the standard deviations of the $M$-sized segments $y_i$.

5) A threshold parameter $\tau > 1$ is defined (typically 1.2< r <1.6).

6) For each segment $y_i$, with $i = 1 \dots N$, the standard deviation $\sigma_{ii}$ is compared to $\tau\sigma_{min}$ with two possible outcomes:

A) if $\sigma_i \leq \tau\sigma_{min}$, the elements of $x^b$ comprised between $x^b[(i - 1)M]$ and $x^b[iM]$ are filled with the average value $\overline{y}_i$

B) if $\sigma_{ii} > \tau\sigma_{min}$, $y_i$ is subdivided into 10 samples, $z_k^i \, (k = 1 \dots 10)$ of length M/10. The elements of $x^b$ comprised between $x^b[(i - 1)M + M(k - 1)/10]$ and $x^b[(i - 1)M + Mk/10]$ are filled with the average value of the $z_k^i$ segment, $\overline{z}_k^i$.

7) The baseline is finally completed by filling the last $W - NM$ elements of $x^b$ with the mean value of the last $W - NM$ elements of $x^f$,

The baseline trace $x^b$ is extremely important because it makes it possible to track changes in the size of the nanopore over time.

<u>Event detection.</u> After determining the baseline trace, the normalized ($\Delta I/I_0$) trace, $x^n$ is calculated as $\chi^n = (\chi^f - \chi^b)/\chi^b$ and processed with the following threshold-based event detection algorithm:

1) The trace $x^n$ is subdivided into $F + 1$ segments, $F$ containing 6 samples each and one containing the remaining $W$-

*FG* samples.

2) For each $G$-sized segment $q_i$, with $i = 1 \dots F$, the mean $\bar{q}_i$ and the standard deviation $\sigma_i$ are computed. Two thresholds are calculated, $T_s = \bar{q} + u\sigma_i$, where $u > 1$ (typically $u > 4$), and $T_e = \bar{q} + \sigma_i$.

3) A preliminary search is performed to identify at which position in $q_i$. the signal first exceeds $T_g$ ($e_g$, event start) and then falls below $T_e$ ($e_g$, event end). For each event, the end and start position of the events in the trace $x^n$ are calculated to include part of the baseline as $E_g = e_g + (i - 1)G - 40$ and $E_e = e_e + (i - 1)G + 40$, and stored (extended events).

4) The detected events are subsequently refined, as a simple threshold-based search could fragment a single translocation into multiple short events, or interpret noise spikes as translocation events:

A) All samples in $x^n$ with value smaller than $T_s$ are stored in an array $w$ of size $R$.

B) A matrix $B_{(R-Q)\times Q}$ is constructed by stacking Q delayed copies of $w$, such that the $j^{th}$ column of $B$ contains the elements of w comprised between $w[j]$ and $w[R - Q + j]$.

C) The standard deviation of each row of $B$ is calculated, and the minimum value obtained is defined $\sigma_0$.

D) Each extended event is fitted using two gaussian peaks having the same standard deviation $\sigma_0$ and centered at positions $L_0$ and $L_1 (L_1 > L_0)$. $L_0$ and $L_1$ represent an estimate of the baseline and of the event amplitude, respectively.

E) The event is fitted with a 2-level step-fitting algorithm using $L_0$ and $L_1$ as levels.

F) The portion of the event which is best fitted by $L_1$ is considered as the refined event. If the duration of the event exceeds a user-defined minimum duration, the event is accepted and stored.

<u>Event analysis.</u> The refined translocation events are analyzed to determine the minimum and the maximum intensity values ($dl_{min}, dl_{max}$), which are needed to determine the volume ($\Lambda$) and the ellipsoid-equivalent shape (m) of the translocating particles. Since the measurements are affected by noise, the absolute maximum and minimum of the event trace are not good estimates of $dl_{min}$ and $dl_{max}$. Instead, we developed the following method:

1) A matrix $B_{(R-Q)\times Q}$ is constructed as described above. The $20^{th}$ and the $80^{th}$ percentile of each row of $B$ are calculated and stored in two arrays, $P_{20}$ and $P_{80}$, with sizes $(R - Q)$.

2) The minimum value of the array obtained from the elementwise difference $P_{80} - P_{20}$ is labelled $dl_0$.

3) The $20^{th}$ and the $80^{th}$ percentile of each event, $dl_{20}$ and $dl_{80}$ is computed.

4) $dl_{min}$ and $dl_{max}$ are calculated as $dl_{min} = dl_{20} + dl_0 / 2$ and $dl_{max} = dl_{80} - dl_0 / 2$.

5) $dl_{min}$ and $dl_{max}$ are used to compute $\Lambda$ and $m$ as described in references 13,40.

*Calculation of reference values for $\Lambda$ and m*

Reference values for $\Lambda$ and $m$ can be calculated using the following procedure:

1) The atomic coordinates of the target protein are extracted from the .PDB file

2) A grid with lattice dimension I is constructed to fully incorporate the protein.

3) The number of cells $N_C$ in the grid containing at least one atom of the protein are counted.

4) The volume of the protein is determined as $\Lambda = N_C l^3$.

5) All coordinates are rescaled by subtracting their average values ( $x_i' = x_i - \bar{x}$ ,

$y_i' = y_i - \bar{y}, z_i' = z_i - \bar{z}$ ).

6) The equation of an ellipsoid of rotation with volume $\Lambda_r$ is calculated as a function of the axis ratio $m_r$, of the coordinates of the center ($x_0, y_0$ and $z_0$) and the three Euler angles around the ellipsoid axis ($\alpha, \beta$ and $\gamma$).

7) $x_0, y_0$ and $z_0, \alpha, \beta, \gamma$ and $m_r$ are optimized using a non-linear fitting procedure based on a search method to maximize the number of atoms of the protein enclosed within the ellipsoid.

## Examples

### Example 1 - Material & methods

*Complement component 9 (C9) assemblies:* The formation of polyC9 rings requires C9 concentrations of approximately 1.5 mg/ml. Commercial complement component 9 solution, which contains 1 mg/ml C9 in phosphate buffer (PBS, pH 7.2), was concentrated via a buffer exchanged procedure using a 30 kDa MWCO (Amicon) protein spin filter to obtain a final concentration of 2 mg/ml C9 in a 10 mM HEPES pH 7.5, 50 mM NaCl buffer (polyC9 buffer). The buffer exchange was performed using a centrifuge (Eppendorf® Centrifuge 5427R) at 6,500 rpm for 5 min, repeated 3 times. The final concentration of C9 was verified using a spectrophotometer (NanoDrop) with Proteins A280 software. A stock solution of amphipol (0.02 mg/ml) was prepared by dissolving the dry powder in polyC9 buffer. The stock solution of Amphipol (0.02 mg/ml) was added in a 9:1 volume ratio to the C9 solution. 10 $\mu$l of the obtained mixture was transferred into a 0.5 ml Eppendorf tube and incubated overnight at 37 °C in a thermostatic mixer (ThermoMixer). After incubation, the sample was kept at 4 °C for later use.

*Crosslinked-polyC9:* A stock solution of disuccinimidyl suberate (DSS) (50 mM) was prepared by dissolving the dry powder in dimethyl sulfoxide (DMSO). 0.1 $\mu$l of the DSS solution was added to 0.99 $\mu$l of the incubated C9 solution (20 DSS equivalents with respect to C9), and the mixture was incubated at room temperature for 1 h. The reaction was quenched by adding 0.25 $\mu$l Tris-HCl (1 M) to get a final concentration of 20 mM. After 15 minutes of incubation at room temperature, the mixture was purified by classic dialysis. Before the dialysis, the 10 kDa molecular weight cut off (10 kDa MWCO) was equilibrated for 1 h in 100 ml of 10 mM HEPES pH 7.5, 50 mM NaCl buffer with 250 ml beaker using stirrer bar at 250 rpm. The obtained mixture was then added into equilibrated 10 kDa MWCO. The dialysis was carried out for 3 h at 250 rpm. The sample was kept at 4°C for later use.

*Planar lipid bilayer recordings:* We carried out all electrical recording using an integrated chip-based, four channel parallel bilayer recording setup (Orbit Mini; Nanion Technologies) with multielectrode cavity-array (MECA) chips (Ionera, Germany). 1,2-diphytanoyl-sn-glycero-3-phosphocholine (DiphyPC) powder was dissolved in octane at a final concentration of 15 mg/ml. This solution was used to form lipid membranes as explained in the next section. The recording buffers were used to form the membrane depending on the objective of individual experiment. For insertion of polyC9 at varying NaCl concentration, the recording buffers consist of 10 mM HEPES buffer (pH 7.5) with 0.02 mg/ml amphipol an NaCl concentrations ranging from 50 mM to 1 M. For insertion of polyC9 at varying pH, the recording buffers contain of 1 M NaCl, 0.002 mg/ml amphipol and various buffers (10 mM) depending on the target pH (sodium acetate for pH = 5.5, HEPES for pH = 6.5 and 7.5, N-(1,1-dimethyl-2-hydroxyethyl)-3-amino-2-hydroxypropanesulfonic acid (AMPSO) for pH = 8.5). We filtered the recording buffers with 0.2 $\mu$m poly(ether sulfone) filters (VWR, Radnor, PA, USA) before use. The top (cis) chamber is connected electrically to the ground, while the wells below each of the four lipid membranes, generally indicated as trans chambers, contain the working electrodes.

In a typical experiment, the incubated C9 solution is added to the cis chamber until a single pore opens in at least one of the four membranes (see Membrane insertion of polyC9 section for details). The analyte to be characterized via translocation experiments is subsequently added in the cis compartment. We carried out all experiments at 25 °C. The recordings were performed with the EDR5 software (Elements) selecting a sampling rate of 200 kHz and 20 nA range. The recorded current traces were analyzed using Clampfit 11.2 (Molecular Devices) and a custom-built data analysis package in Python, described in the section *Data analysis algorithm.*

*Formation of the membrane:* We used MECA chips bearing four holes with diameter of 50 $\mu$m to support membrane formation. The membranes were formed using the technique described by Wang et al[38]. Briefly, we added the recording buffer (150 $\mu$l) to the cis compartment and all four channels were wetted by applying a gentle pressure on the holes using a syringe plunger. The bilayers were then formed by painting the lipid solution (2 -3 $\mu$L) closed to the cavity of the chips. We confirmed good quality of the membrane by measuring a baseline current (-0.3 < I < 0.3 nA) and capacitance of 7 $\pm$ 2 nF. The recording software automatically estimates the membrane capacitance by analyzing the current response to an applied triangular potential. Prior the polyC9 addition, we checked the stability of the bilayer (absence of leak currents, expected noise level) by applying transmembrane voltages of up to 100 mV for 1 min at both polarities.

*Membrane insertion of polyC9:* We carried out all polyC9 insertion experiment by adding 2 ml of the polyC9 solution into the cis chamber to obtain a 92 nM final (monomer equivalent) concentration of C9. An applied potential of +100mV was maintained until a single step current jump was observed, indicating successful incorporation of a polyC9 ring. We calculate the inner diameter and the number of C9 monomers forming the ring form the pore conductance. The pore conductance was obtained from the difference between baseline and open-state pore currents. This diameter of the pore was estimated using the equation proposed by Cruickshank et al[39], which assumes the pore to be perfectly cylindrical and accounts for both the channel and the access resistances:

$$d_{\mathrm{p}} = \frac{\rho_{\mathrm{el}}\,G}{\pi} \times \left( \frac{\pi}{2} + \sqrt{\frac{\pi^2}{4} + \frac{4\pi l_{\mathrm{p}}}{\rho_{el}\,G}} \right) \qquad (1)$$

In equation 1, $d_{\mathrm{p}}$[m] is inner diameter of nanopore, $l_{\mathrm{P}}$[m] is the pore length, $\rho_{\mathrm{el}}$[$\Omega \cdot$m]is the electrical resistivity of the electrolyte buffer and $G$ [S] is the conductance of the pore. We estimated the channel length $l_{\mathrm{p}}$ = 13$\cdot$10$^{-9}$ m from the crystal structure of polyC9 (5FMW). We then calculated the number of C9 monomers composing the nanopore using a previously published geometric model[11].

*Proteins translocation:* After formation of a stable C9 nanopore, a stepwise potential sweep from -100 mV to +100 mV was performed to ensure the pore stability and to measure the offset current at 0 applied potential. Subsequently, 5 $\mu$l of analyte solutions at a concentration of 1mg/ml are added into the cis compartment. Translocation of the analyte is promoted by applying constant potentials of either +100 or -100 mV across the nanopore. Every 5 minutes, the potential sweep is repeated to verify the stability of the nanopore and to measure the off-set current. We filtered the acquired ubiquitin and lysozyme data with a Gaussian low-pass filter at a cut-off frequency of 10 kHz for ubiquitin and lysozyme and 20 kHz for eGFP, CA, and FAB. We performed a threshold-search (5 x the standard deviation of the baseline current) for resistive pulses within the current recording using the procedure described in *Data analysis software.*

*Gel electrophoresis:* In a typical gel electrophoresis experiment, 5 $\mu$l of 2 mg/ml polyC9 was mixed with LDS Sample Loading Buffer before adding the sample into the wells. The electrophoresis was carried out on 4 - 15% Mini-PROTEAN® TGX™ Precast Protein Gels under 250 mV applied potential for 40 min. Subsequently, the gel was immersed into the ReadyBlue™ Protein Gel Stain overnight. The gel was finally washed with water to eliminate excess stain solution. The marker used to estimate molecular weight was SigmaMarker™, which has a wide range of molecular weight. 6.5 - 200 kDa (molecular weight of 6.5, 14.2, 20, 24, 29, 36, 45, 55, 66, 97, 116, 200 kDa).

*Transmission electron microscopy (TEM):* We used an oxygen plasma cleaner to clean carbon-coated 300-mesh copper grids (Electron Microscopy Sciences, Hatfield) for 5 s to ensure that the grid was cleaned and had a negative charge. Then, 5 $\mu$l of polyC9 sample (~2 mg/ml concentration) was drop-cast on the carbon-coated copper grid. The grid was then stained with 3 $\mu$l of 2 % v/v aqueous uranyl acted solution and incubated for 2 min. Excess stain was blotted off with a filter paper and dried at room temperature for 30 minutes. The TEM analysis was performed using an electron microscope (FEI Tecnai G2) at an operating voltage of 120 kV. We used imaged for measuring inner diameter of C9 nanopore.

*Mass photometry:* We acquired all the mass photometry data using a TwoMP mass photometer (Refeyn Ltd, Oxford,UK) using a protocol similar to that described previously with slight modifications. Briefly, microscope coverslips (24 $\times$ 50 mm Thorlabs, Cat. No. CG15KH1) were cleaned using isopropanol followed by pure water repeatedly, and then dried by nitrogen flow. We have used reusable self-adhesive silicone culture wells to keep the sample droplet in shape (Cat. No. GBL103250). The gasket was placed in the center of the clean coverslip and ensured that it was fixed tightly by applying light pressure with a clean tweezer. A droplet of immersion oil was applied to the objective and the prepared coverslip was mounted so that no bubbles are in the immersion oil. Small magnets were placed to fix the coverslips. To study the mass distribution of polyC9, 18 $\mu$l of polyC9 buffer was added, and then the focus was adjusted. We diluted polyC9 up to a concentration of 0.2 mg/ml in polyC9 buffer immediately prior to experiments. 2 $\mu$l (0.2 mg/ml) solution was added to the buffer droplet for mass photometry measurements. The movie was recorded for 180s. The mass photometry calibration was analyzed using DiscoverMP, where mean contrast values of all peaks were determined in the software using Gaussian fitting. Contrast-to-mass calibration was conducted using NativeMark™ Unstained Protein Standard of known molecular weight, including Bovine serum albumin, Lactate dehydrogenase, B-phycoerythrin, Apoferritin, Apoferritin oligomer, IgM pentamer, and IgM hexamer. The mean contrast values were plotted against the known molecular weight of proteins.

## *Example 3 - Complement component 9 nanopores*

When a pathogen is detected by the immune system, enzyme activation is triggered in a cascade leading to the assembly of five proteins into a membrane attack complex (MAC) capable of forming lethal pores in the plasma membrane of the pathogen. The main component of the MAC, which exhibit a tubular shape with an internal diameter of 12 nm in and a length of 16 nm, are the C5b-8 protein complex and multiple copies of complement component 9 (C9). Although in biological systems C9 assembly is triggered by the formation and membrane insertion of the C5b-8 complex, C9 self-assembly can also occur spontaneously in solution under certain conditions, resulting in tubular complexes with amphiphilic properties. The structure of the assembled C9 (polyC9) has recently been elucidated via cryogenic electron microscopy (cryo-EM), which unveiled a symmetrical arrangement of 22 C9 units and showed a ring-like structure with internal cross-sectional diameter of 12 nm and length of 16 nm like the MAC (Figure 1a).

The assembly of C9 has been investigated under various environmental conditions and in the presence of additives. It is however not reported how these conditions impact the ability of polyC9 to insert into phospholipid bilayer membranes and form functional transmembrane pores. We incubated C9 (28 $\mu$M) in a 50 mM NaCl solution containing 0.002 mg/ml of the surfactant Amphipol and 10mM HEPES buffer (pH = 7.5) and we characterized the resulting polyC9 rings by gel electrophoresis (SDS-page), transmission electron microscopy (TEM), and mass photometry. Figure 1b, lane 2 reveals that incubated C9 consist of two populations. The low molecular weight band (molar mass value of approximately 60 kDa) is consistent with the C9 monomer (lane 1), showing that not all C9 assembled into rings. The high molecular weight band appears at the top of the lane and falls outside the marker range (lane M). We attribute this band to polyC9, although it is not possible to reliably determine its molecular weight. Established single-particle methods, including negative staining TEM imaging (Figure 2) and mass photometry (Figure 3), confirmed the presence of C9 assemblies. The TEM analysis reveals the characteristic appearance of the polyC9 ring with an average inner diameter of 11.2 $\pm$ 1.9 nm (Figure 2c). Mass photometry demonstrates that the dominating polyC9 population has an average molecular weight of 1.6 MDa, approximately 22 times the monomer mass. Smaller aggregates are also present such as dimers, trimers and higher order assemblies (Figure 3). These results agree with previous cryo-EM studies reporting that C9 assembly consists of 22 C9 monomers with an inner diameter of 12 nm and a mass of 1.6 MDa.

Subsequently, we explored the ability of polyC9 to form functional pores into lipid membranes under different conditions (See method section Planar lipid bilayer recordings). Figure 4 displays electrical recordings of polyC9 single-pore insertions, where the magnitude of the current jump caused by the ring insertion enables the estimation of the nanopore inner diameter (See method section Formation of polyC9).

PolyC9 formed in 50 mM NaCl, 0.002mg/ml Amphipol, buffered at pH 7.5 with 10 mM HEPES yields pores with an average diameter of 10.1 $\pm$ 3.4 nm in recording buffers containing 50 mM NaCl, buffered at pH 7.5 with 10 mM HEPES (Figure 4a, b). These pores remain stable for over 30 minutes after formation. Crosslinking of polyC9 after incubation (see method section Crosslinked-polyC9) does not affect the ability of polyC9 to form functional pores (Figure 4c, d) nor the pore stability and the average inner diameter (9.8 $\pm$ 2.1 nm.)

At 1M NaCl (Figure 4e, f), polyC9 formed transmembrane pores only in 3% of the attempts, as opposed to an 80% success rate observed at 50 mM NaCl. The stability of the pore also decreased drastically and the open pore state duration reduced to 3 minutes on average.

Typically, the analysis of proteins with nanopore techniques is performed at high salt concentrations to maximize the signal-to-noise ratio. Unfortunately, native C9 assemblies form unstable pores at 1M salt concentrations. We used the commercial polymeric surfactant amphipol to promote the formation of polyC9 nanopores and their insertion into phospholipid membranes (see method section Planar lipid bilayer recordings). In the presence of Amphipol, polyC9 forms nanopores with a success rate of 70% while the open pore state duration increases on average to more than 30 minutes. This result is in contrast with previous accounts reporting that the presence of surfactants such as sodium deoxycholate and sodium dodecyl sulfate can destabilize the lipid membrane with negative effects on the nanopore stability.

Figure 5a depicts the current-voltage (I-V) curve of C9 nanopore and crosslink- C9 nanopore at different salt concentrations. In all cases the C9 nanopore displays ohmic behavior (slope of 2.2 pA.mV$^{-1}$ and Pearson's $r$ = 0.994 for C9 nanopore in 50mM NaCl without Amphipol; slope of 2.1 pA.mV$^{-1}$ and $r$ = 0.995 for crosslink- C9 nanopore in 50mM NaCl without Amphipol; slope of 21.3 pA.mV$^{-1}$ and $r$ = 0.999 for C9 nanopore in 1

M NaCl with Amphipol) indicating that the pore lumen is cylindrical, insensitive to voltage polarity and stable over a wide range of applied potentials.

We defined a noise parameter, calculated as the standard deviation of the open pore current divided by the standard deviation of the pre-insertion baseline current, and we compared the power spectral densities (PSD) of the recordings before and after nanopore insertion to assess the stability of the C9 nanopore under different conditions. At 50 mM NaCl both C9 nanopore and cross-linked C9 nanopore exhibit an average noise parameter close to 1, indicating that the formation of nanopores under these conditions does not lead to significant increase of the electrical noise. The only notable difference is that cross-linked C9 nanopore shows a larger standard deviation, suggesting that crosslinking can be beneficial for noise reduction but inhomogeneous at the single particle level. Insertion of polyC9 at 1 M NaCl results in a significant increase of the signal noise (Figure 5b, and c). This increase mostly interests the low-frequency component of the noise (Figure 5 c), hinting at fluctuations of the polyC9 structure. The fact that these fluctuations manifest only at high salt concentration suggests that the C9 nanopore is stabilized by electrostatic attraction. With increasing salt concentration, charge screening reduces the range of this attraction causing structural instabilities. This hypothesis is consistent with the structure of assembled C9, where oppositely charged residues are distributed at opposite sides of the protein stabilizing the interaction of adjacent units. The addition of Amphipol at 1M NaCl (Figure 5 b, and d) drastically improves the probability of insertion and the lifetime of C9 nanopore, yet it favors only a slight reduction of the current noise. This leads to the conclusion that Amphipol mediates the interaction between C9 and the phospholipid bilayer stabilizing the membrane-pore adduct rather than the C9 nanopore itself.

### Example 4 - C9 nanopore devices yield the volume and the shape of translocating analytes

While biological nanopores have been used heretofore for protein sensing or DNA sequencing, C9 nanopore can be employed to determine the volume and the shape of freely translocating analytes at nanomolar concentrations. Figure 6 demonstrates the schematic representation of a recording setup for single molecule analysis with C9 nanopore embedded in a planar lipid bilayer. To demonstrate this capability, we chose five different proteins - ubiquitin, lysozyme, carbonic anhydrase (CA), enhanced green fluorescent protein (eGFP), and human IgG Fab fragment (FAB) - that differ in molecular weight (M.W. = 8.6 to 50 kDa), volume ($\Lambda$ =13.3 to 64.7 nm$^3$), and length-to-diameter ratio (m = 0.58 to 1.59) showed in Figure 7. For a protein with volume $\Lambda$, the expected length-to-diameter ratio m is defined as the m value of the ellipsoid of rotation with volume $\Lambda$ that contains the largest portion of the protein.

We determined volume and shape of the selected proteins using the theory described by Golibersuch and others, which relates the amplitude of the resistive pulse to the volume of particle and the shape of the signal to the particle's shape (see method section Protein translocations). According to previous studies, proteins have to sample many orientations while translocating through the nanopore to accurately determine m and $\Lambda$. To ensure that this condition is met, we analyzed data with a dwell time exceeding 450 $\mu$s in the case of ubiquitin and lysozyme (the data was digitally low-pass filtered at 10 kHz) and a dwell time exceeding 300 $\mu$s in the case of CA, eGFP, and FAB (the data was digitally low-pass filtered at 20 kHz). The data were analyzed using the algorithm describe in the methods section Data analysis algorithm. Figure 8 shows the distribution of experimental volumes and length-to-diameter ratios compared with reference values for each protein, illustrating that this analysis yields excellent estimates of $\Lambda$ and m (Figure 9).

***Example 5 - C9 nanopore devices with tunable selectivity.***

The translocation of proteins through nanopores takes place under the influence of two main forces: electrophoresis and hydrodynamic drag by electroosmotic flow. These phenomena depend on the applied potential V, on the charge of the proteins and on the charge of the inner surface of the nanopore. The charge state of a protein, as well as that of a surface, depend on the nature of the charged groups and on the pH of the medium. We hypothesize that tuning pH and applied potential make it possible to control the balance between electrophoresis and electroosmotic flow thereby favoring or disfavoring the translocation of a certain analyte on demand. First, we verified that polyC9 form stable and functional nanopores at pH ranging from 5.5 to 8.5. We then selected ubiquitin and eGFP, that differ in size and isoelectric point (pI), and we performed translocation experiments at various pH and applied potentials (Figure 10 and 11). We added Ubiquitin and eGFP in one of the two electrolyte-filled chambers separated by the nanopore, which was electrically grounded by means of an Ag/AgCl electrode. We applied positive or negative potentials (V = $\pm$100 mV) in the opposite chamber by means of another Ag/AgCl electrode. Figure 10 and 11 show that translocation can occur with both positive and negative potentials, confirming that both electrophoresis and electroosmotic flow are at play. As hypothesized, we did not observe translocations for certain combinations of potentials and pH, demonstrating that the nanopore selectivity can be tuned on demand by varying these two parameters.

Additionally, the translocation frequency, as well as the translocation frequencies at negative and positive applied potentials, can be modified on demand by tuning pH, as shown in Figure 12 and Table 1. Finally, Figure 13 demonstrates that C9 nanopore enable the determination of $\Lambda$ and m at various pH with negligible effects on the accuracy of the measurement.

**Table 1** Translocation event rates at varying pH

| Protein | | | pH 5.5 | pH 6.5 | pH 7.5 | pH 8.5 |
|---|---|---|---|---|---|---|
| Ubiquiti n | Estimated net charge[a] | | 1.7 | 0.6 | -0.2 | -1 |
| | Event rate (s$^{-1}$) | V = - 100mV | 0.4$\pm$0.4 | 0.7$\pm$0.1 | 0.9$\pm$1.1 | No translocation |
| | | V=+100mV | 0.3$\pm$0.3 | 0.8 | 0.2$\pm$0.1 | No translocation |
| | Event rate ratio[b] | | 1.6 | 0.9 | 4.5 | - |
| eGFP | Estimated net charge[a] | | 14.7 | 3.2 | -6.5 | -10.5 |
| | Event rate (s$^{-1}$) | V = - 100mV | 2.3 $\pm$ 1.1 | 0.6 $\pm$ 0.4 | 1.7 $\pm$ 0.8 | 1.2 $\pm$ 1.4 |
| | | V=+100mV | 0.7 $\pm$ 0.6 | 0.9 $\pm$ 0.7 | 0.5 $\pm$ 0.1 | 0.2 $\pm$ 0.6 |
| | Event rate ratio[b] | 3.1 | 0.7 | 3.3 | 7.1 | |
| *a: The net charge estimation is calculated based on the amino acid composition using https://protcalc.sourceforge.net/.* *b: The event rate ratio is the ratio between the event rate under applied negative bias and the event rate under applied positive bias.* | | | | | | |

**Claims**

1. A nanopore-based sensing device comprising at least one membrane, the membrane comprising at least one nanopore comprising complement component 9 (C9) monomers.

2. The nanopore-based sensing device according to claim 1, wherein the nanopore has diameter in the range of 5 to 15 nm, preferably 10 nm, and/or an effective length in the range of 3.5 to 20 nm, preferably 13 nm.

3. The nanopore-based sensing device according to claim 1 or 2, wherein the device is capable of sensing proteins, macromolecules, nanoparticles and/or assemblies of nanoparticles.

4. The nanopore-based sensing device according to any of claims 1 to 3, wherein the nanopore comprises between 10 to 35 C9 monomers, preferably 22 C9 monomers.

5. The nanopore-based sensing device according to any of claims 1 to 4, wherein at least one C9 monomer is modified compared to a naturally occurring monomer, the modification being an amino acid exchange, deletion and/or insertion and/or a chemical derivatization of an amino acid.

6. The nanopore-based sensing device according to claim 5, wherein at least one C9 monomer is modified by replacing at least one cysteine residue with another amino acid and/or by adding a functional group to at least one cysteine residue.

7. The nanopore-based sensing device according to 5 or 6, wherein the at least one C9 monomer is modified by replacing at least one amino acid carrying a charge with an amino acid carrying the opposite or no charge.

8. The nanopore-based sensing device according to 6 or 7, wherein at least one amino acid selected from the group comprising residues 183 to 281 and 330 to 408 of SEQ ID NO: 1 and/or at least one amino acid selected from the group comprising residues 18 to 70, 80 to 134,148 to 182 and 485 to 523 of SEQ ID NO: 1 is modified.

9. The nanopore-based sensing device according to claim 5, wherein the first 16 amino acids of the N-terminus of C9 are deleted and/or the C9 monomers are crosslinked with each other.

10. The nanopore-based sensing device according to any of claims 1 to 9, wherein the at least one nanopore is stabilized with a polymeric surfactant and/or has been assembled in the presence of a polymeric surfactant, wherein the polymeric surfactant is preferably an amphipol.

11. The nanopore-based sensing device according to any of claims 1 to 10, wherein the sensing properties of the device are adjustable via the applied electric field, properties of the ions in the electrolyte solution, concentration of ions in the electrolye solution, and/or the pH of the electrolyte solution.

12. A kit for assembling a nanopore-based sensing device, the kit comprising

   • a device comprising at least one membrane,
   • pre-conditioned C9 monomers and/or at least one pre-assembled nanopore consisting of C9 monomers,
   • means to facilitate assembly of the pre-conditioned C9 monomers into a nanopore and/or insertion of the assembled or pre-assembled nanopore into the membrane.

13. The kit according to claim 12, wherein the means to facilitate assembly of the pre-conditioned C9 monomers into a nanopore and/or insertion of the assembled or pre-assembled nanopore into the membrane comprises a buffer comprising a polymeric surfactant, preferably amphipol.

14. Use of a nanopore consisting of C9 monomers for sensing proteins, macromolecules, nanoparticles and/or assemblies of nanoparticles.

15. Use of a nanopore consisting of complement component 9 monomers for controlling the translocation probability of a molecule or particle of interest.

16. Use of a polymeric surfactant, preferably amphipol, for assembling a nanopore from protein monomers or inserting a pre-assembled nanopore into a membrane.

17. A computer-implemented method of determining the volume and/or shape of an analyte, the method comprising:

   a) importing a nanopore signal recording, wherein the signal is the current through the nanopore in the presence of the analyte, wherein the analyte is able to enter and cross the nanopore (translocate through the nanopore) and wherein a translocation event is the portion of the signal pertaining to the translocation of the analyte;
   b) determining a dynamic baseline trace by processing the imported recording with an algorithm that discriminates between fluctuations of the signal caused by naturally occurring variations of the nanopore size and translocation events and using the dynamic baseline to normalize the imported signal;

c) performing a threshold-based search of the normalized signal to identify translocation events

d) calculating the size of the nanopore at any time point in the recording from the dynamic baseline trace determined in step b);

e) analyzing each translocation event detected in step c) with a percentile-based method that extracts the noise-corrected minimum and maximum amplitudes of the translocation event;

f) determining for each translocation event the volume of the analyte using the minimum and maximum amplitudes calculated in step e) and the actual nanopore size calculated in step d);

g) deriving, for each translocation event, the most probable shape of the analyte from the shape of the amplitude distribution of the translocation event and from the minimum and maximum amplitudes calculated in step e).

a

5.5 nm  10.5 nm

12 nm

b

M   1   2

kDa

200
166
97
66

55
45

36
29

24

20

**Figure 1**

Figure 2

Figure 3

**Figure 4**

Figure 5

Figure 6

Figure 7

Figure 8

Figure 9

Figure 10

Figure 11

Figure 12

Figure 13

NANOPORE DATA ANALYSIS

REFERENCE VALUES

Figure 14

| 18 | SHIDCRMSPWSEWSQCDPCLRQMFRSRSIEVFGQFNGKRCTDAVGDRRQC | 67 |
|---|---|---|
| 68 | VPTEPCEDAEDDCGNDFQCSTGRCIKMRLRCNGDNDCGDFSDEDDCESEP | 117 |
| 118 | RPPCRDRVVEESELARTAGYGINILGMDPLSTPFDNEFYNGLCNRDRDGN | 167 |
| 168 | TLTYYRRPWNVASLIYETKGEKNFRTEHYEEQIEAFKSIIQEKTSNFNAA | 217 |
| 218 | ISLKFTPTETNKAEQCCEETASSISLHGKGSFRFSYSKNETYQLFLSYSS | 266 |
| 267 | KKEKMFLHVKGEIHLGRFVMRNRDVVLTTTFVDDIKALPTTYEKGEYFAF | 316 |
| 317 | LETYGTHYSSSGSLGGLYELIYVLDKASMKRKGVELKDIKRCLGYHLDVS | 366 |
| 367 | LAFSEISVGAEFNKDDCVKRGEGRAVNITSENLIDDVVSLIRGGTRKYAF | 416 |
| 417 | ELKEKLLRGTVIDVTDFVNWASSINDAPVLISQKLSPIYNLVPVKMKNAH | 466 |
| 467 | LKKQNLERAIEDYINEFSVRKCHTCQNGGTVILMDGKCLCACPFKFEGIA | 516 |
| 517 | CEISKQ | 523 |

Figure 15

a

b

c

d

Figure 16

Figure 17

Figure 18

Europäisches
Patentamt

European
Patent Office

Office européen
des brevets

# EUROPEAN SEARCH REPORT

**Application Number**

EP 23 20 6767

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/057432 A1 (OXFORD NANOPORE TECH PLC [GB]; UNIV MONASH [AU]) 13 April 2023 (2023-04-13) * pages 1-4, 36- - page 41; claims 7, 26 * | 1-17 | INV. G01N33/487 C12Q1/6869 C12Q1/6874 |
| X | US 2014/246317 A1 (MAYER MICHAEL [US] ET AL) 4 September 2014 (2014-09-04) | 17 | |
| A | * paragraphs [0039] - [0044], [0099] - [0109] * | 1-16 | |
| A | YING LUN ET AL: "Nanopore-based technologies beyond DNA sequencing", NATURE NANOTECHNOLOGY, [Online] 1 November 2022 (2022-11-01), pages 1136-1146, XP055981713, DOI: 10.1038/s41565-022-01193-2 Retrieved from the Internet: URL:https://www.nature.com/articles/s41565 -022-01193-2.pdf> * the whole document * | 1-17 | |
| A | US 2023/176034 A1 (MAGLIA GIOVANNI [NL] ET AL) 8 June 2023 (2023-06-08) * the whole document * | 1-17 | TECHNICAL FIELDS SEARCHED (IPC) G01N C12Q |

The present search report has been drawn up for all claims

| Place of search | Date of completion of the search | Examiner |
|---|---|---|
| The Hague | 1 August 2024 | Martin, Hazel |

EPO FORM 1503 03.82 (P04C01)

Europäisches
Patentamt
European
Patent Office
Office européen
des brevets

**Application Number**

EP 23 20 6767

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

    1. claims: 1-17

        membrane comprising at least one nanopore comprising complement component 9

    1.1. claim: 16

        use of a polymeric surfactant for assembling a nanopore

    1.2. claim: 17

        computer-implemented method of determining the volume and/or shape of an analyte
                            - - -

Please note that all inventions mentioned under item 1, although not necessarily linked by a common inventive concept, could be searched without effort justifying an additional fee.

## ANNEX TO THE EUROPEAN SEARCH REPORT
## ON EUROPEAN PATENT APPLICATION NO.

EP 23 20 6767

This annex lists the patent family members relating to the patent documents cited in  the above-mentioned European search report.
The members are as contained in the European Patent Office EDP file on
The European Patent Office is in no way liable for these particulars which are merely given for the purpose of information.

01-08-2024

| Patent document cited in search report | | Publication date | Patent family member(s) | | Publication date |
|---|---|---|---|---|---|
| WO 2023057432 | A1 | 13-04-2023 | AU | 2022358916 A1 | 07-03-2024 |
| | | | CA | 3232166 A1 | 13-04-2023 |
| | | | CN | 118056021 A | 17-05-2024 |
| | | | EP | 4413161 A1 | 14-08-2024 |
| | | | KR | 20240068058 A | 17-05-2024 |
| | | | WO | 2023057432 A1 | 13-04-2023 |
| US 2014246317 | A1 | 04-09-2014 | US | 2014246317 A1 | 04-09-2014 |
| | | | US | 2017138898 A1 | 18-05-2017 |
| | | | US | 2019323990 A1 | 24-10-2019 |
| US 2023176034 | A1 | 08-06-2023 | US | 2023176034 A1 | 08-06-2023 |
| | | | WO | 2021182957 A1 | 16-09-2021 |

EPO FORM P0459

For more details about this annex : see Official Journal of the European Patent Office, No. 12/82